# EUROPEAN PATENT APPLICATION

(11) **EP 2 394 573 A1**
(43) Date of publication of application: **14.12.2011**
(21) Application number: 11169220.8
(22) Date of filing: 09.06.2011
(51) Int. Cl.: A61B 5/103, A61B 5/11

(54) **Knee ligament testing device for measuring drawer and rotational laxity**

(30) Priority: 11.06.2010 US 354060 P
(71) Applicant: Arthrex, Inc., Naples, FL 34108-1945 (US)
(72) Inventor: Crabtree, Stephanie, Naples, FL Florida 34109 (US); Karnes, G. Joshua, Estero, FL Florida 33928 (US); Tibone, James E., Los Angeles, CA California 90045 (US); Perez, Arley, Bonita Springs, FL Florida 34134 (US)
(74) Representative: Rosenquist, Per Olof

(57) **Abstract**

A system for determining laxity in an anatomical joint. The system includes a force sensor that measures a force applied to an appendage that is coupled to a joint and a position sensor that determines the amount of movement of the appendage when the force is applied to the appendage. The system also includes a system controller that is connected to both the force sensor and the position sensor and allows communications between the sensors.

## Description

### FIELD OF THE INVENTION

The present invention relates to orthopedic measuring devices and, more particularly, to a device for measuring anterior-posterior force and rotational torque in the knee.

### BACKGROUND OF THE INVENTION

When joints or soft tissues such as tendons or ligaments are injured, testing is necessary to diagnose the extent of the injury, to determine proper treatment and to minimize future damage.

Injuries to the anterior cruciate ligament (ACL) or tendons, ligaments and joints in the knee can result in impaired movement or knee laxity and conditions such as osteoarthritis. In general, there are two types of knee laxity, rotational knee laxity and anterior-posterior knee laxity. Rotational laxity occurs when the ligaments in the knee allow the tibia to over rotate with regard to the femur. Anterior-posterior laxity or drawer laxity occurs when the ligaments in the knee allow the tibia to shift forward and backward with regard to the femur. Reconstruction of the ACL has been shown to decrease both types of knee laxity thereby increasing patient comfort and mobility. Accordingly, it is important to identify the relative movement between the bones connected by the ACL or other ligaments, tendons and joints in the knee to determine if the injury caused rotational and/or anterior-posterior knee laxity.

Techniques have been developed to diagnose and measure rotational knee laxity as well as anterior-posterior knee laxity. Diagnoses of both types of laxity helps to determine the type of reconstruction that needs to be performed to better restore normal knee kinematics in six degrees of freedom.

Techniques to diagnose rotational and drawer laxity that do not employ instruments involve physical examination governed by protocols and developed tests. The disadvantages of physical examination are its subjectivity and dependence on the examiner's skill and expertise. Several commercially available arthrometers that measure anterior-posterior knee laxity exist. While some of these arthrometers can measure rotational knee laxity, these devices are either not portable or have certain limitations with respect to measurement. For example, U.S. Pat. No. 4,583,555 issued to Malcom et al., discloses a device for testing anterior and posterior cruciate ligaments of the knee by measuring drawer force applied to the patient. By virtue of its design, the device allows, however, for measuring of only anterior-posterior knee laxity and cannot be employed to measure any other direction of force.

Accordingly, there is a need for an arthormeter that measures both anterior-posterior knee laxity and rotational knee laxity in a portable format.

### SUMMARY OF THE INVENTION

The present invention provides a system for measuring both rotational laxity and posterior-anterior (i.e. drawer) laxity of a joint. The system uses sensors to measure drawer force and rotational torque applied to the joint. As the forces are applied to the joint separately, or in combination, at least one additional sensor is used to measure displacement and angular rotation of the joint. These measurements are captured, saved, and displayed. The forces and measurement results may be displayed in real time.

The system may include at least one force sensor for measuring an amount of force applied to an appendage coupled to the joint, at least one movement sensor for measuring movement in the appendage when the force is applied to the appendage, and a system controller connected to the force sensor and movement sensor, wherein the force sensor and movement sensor communicate through the system controller.

In one embodiment, the at least one force sensor is a drawer force sensor for determining an amount of drawer force applied to the appendage of the joint. The system may also include a rotational force sensor for determining an amount of rotational force applied to the appendage of the joint. Furthermore, the joint may be a shoulder joint or knee joint and include a boot that houses a foot and lower leg of a patient.

In another embodiment, the at least one movement sensor may be removably coupled to the boot, may detect movement in six degrees of freedom and may only detect movement when the force applied to the appendage, as determined by the force sensor, exceeds a threshold.

Also described herein is a method for determining laxity in a joint. The method includes measuring a force applied to an appendage coupled to the joint using a force sensor, measuring a location of the appendage using a movement sensor when the applied force is greater than an specified force threshold, and determining movement of the appendage using the measured location of the appendage. The method may also include displaying the applied force and the movement of the appendage on a display.

In one embodiment, the step of measuring the applied force measures a rotational force applied to the appendage using a rotational force sensor. In another embodiment, the step of measuring the applied force measures a drawer force applied to the appendage using a drawer force sensor. The method may also include setting the predetermined force threshold when the applied force is the rotational force or setting the predetermined force threshold when the applied force is the drawer force.

The method may further include measuring a rotational force applied to the appendage using a rotational force sensor wherein the step of measuring the force location of the appendage using a movement sensor occurs when the applied drawer force is greater than an applied drawer force threshold. Additionally, the method may include measuring a rotational force applied to the appendage of the joint using a rotational force sensor wherein the step of measuring the force location of the appendage using a movement sensor occurs when the applied rotational force is greater than an applied rotational force threshold. Furthermore, the method may include measuring a rotational force applied to the appendage of the joint using a rotational force sensor wherein the step of measuring the force location of the appendage using a movement sensor occurs when the applied drawer force and the applied rotational force is greater than an applied drawer force threshold and an applied rotational force threshold respectively.

In one embodiment, the method may include measuring a baseline location of the appendage, wherein the step of determining movement of the appendage uses the force location and the baseline location and measuring a baseline force on the force sensor and determining an actual force applied to the appendage using the applied force and the baseline force. Furthermore, the steps of measuring the force applied to the appendage, measuring the location of the appendage, and determining movement of the appendage may be repeated at least twice and averages of the twice measured applied force and movement of the appendage may be calculated.

In another embodiment, the method may include calculating a velocity of the movement sensor when the force is applied to the joint, where the velocity is an angular or linear velocity of the movement sensor. In yet another embodiment, the method may include measuring a location of a second appendage coupled to the joint using a second movement sensor when the applied force is greater than an predetermined force threshold and determining movement of the appendage using the measured location of the appendage and the measured location of the second appendage. Furthermore, the method may include calculating a flexion angle between the appendage and a second appendage coupled to the joint.

These and other features and advantages will be more apparent from the following detailed description that is provided in connection with the accompanying drawings and illustrated exemplary embodiments.

### BRIEF DESCRIPTION OF THE DRAWINGS

Figure 1 illustrates a system for determining rotational and anterior-posterior laxity of a joint according to an exemplary embodiment.

Figure 2 illustrates a side view of a boot in the system of Figure 1.

Figure 3 illustrates another system for determining rotational and anterior-posterior laxity of a joint according to an exemplary embodiment.

Figure 4 illustrates a side view of a boot in the system of Figure 3.

Figure 5 illustrates a back view of the boot in the system of Figure 3.

Figure 6 is a flowchart of a process for measuring laxity in the anterior-posterior and rotational directions according to an exemplary embodiment.

Figure 7 is a flowchart of a process for measuring laxity in the anterior-posterior and rotational directions according to an exemplary embodiment.

Figure 8 illustrates a display screen showing forces and laxity readings according to an exemplary embodiment.

Figure 9 illustrates another display screen showing forces and laxity readings according to an exemplary embodiment.

### DETAILED DESCRIPTION OF THE PREFERRED EMBODIMENTS

In the following detailed description, reference is made to the accompanying drawings which form a part hereof, and which illustrate specific embodiments. These embodiments are described in sufficient detail to enable those of ordinary skill in the art to make and use them. It is also understood that structural, logical, or procedural changes may be made to the specific embodiments disclosed herein.

The system of the present invention can be used to measure both rotational laxity and posterior-anterior (i.e. drawer) laxity of a joint. The system uses various sensors to measure either the rotational and posterior-anterior laxity separately or at the same time.

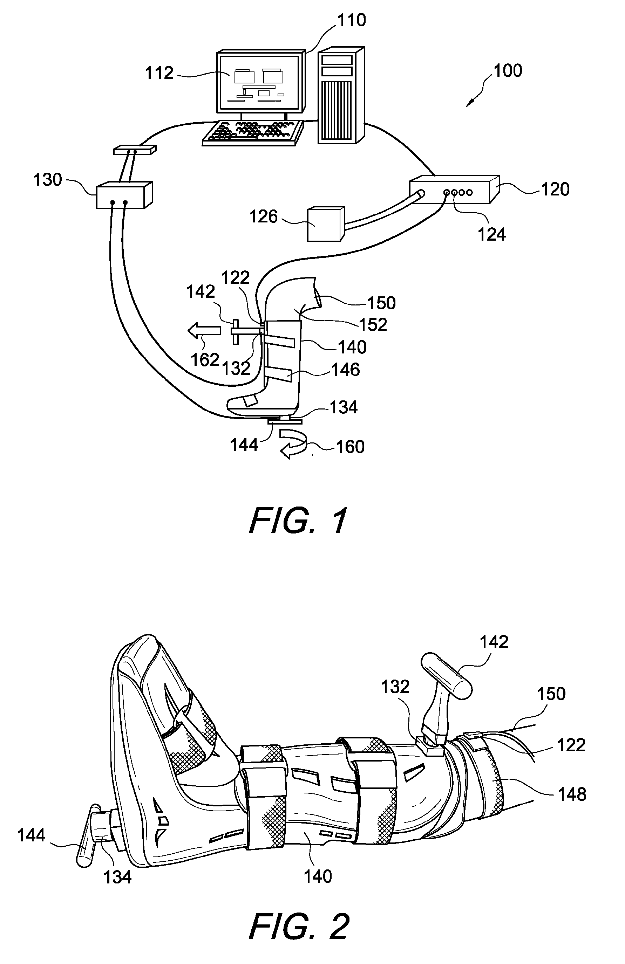

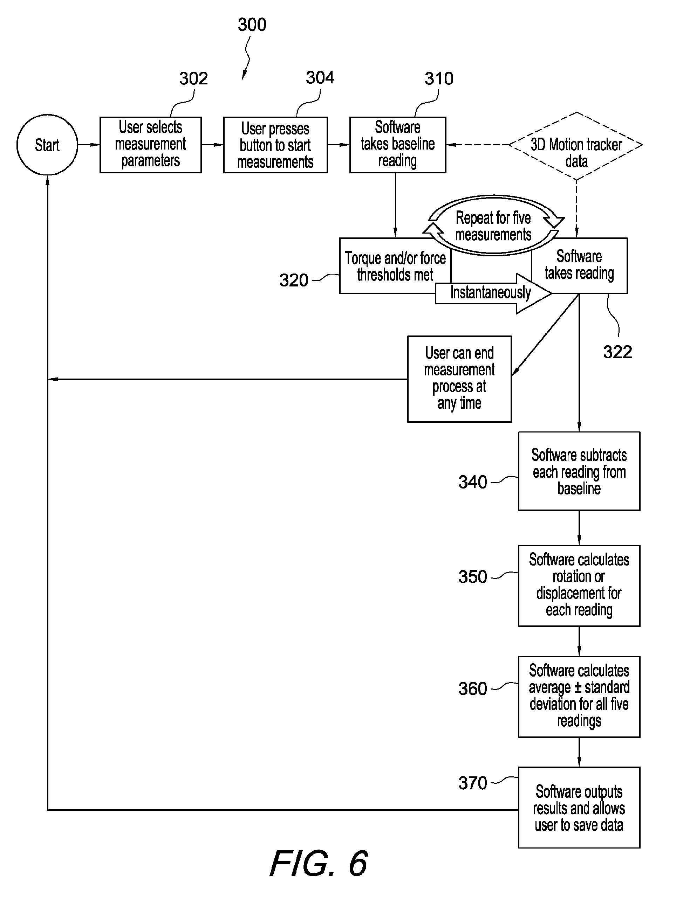

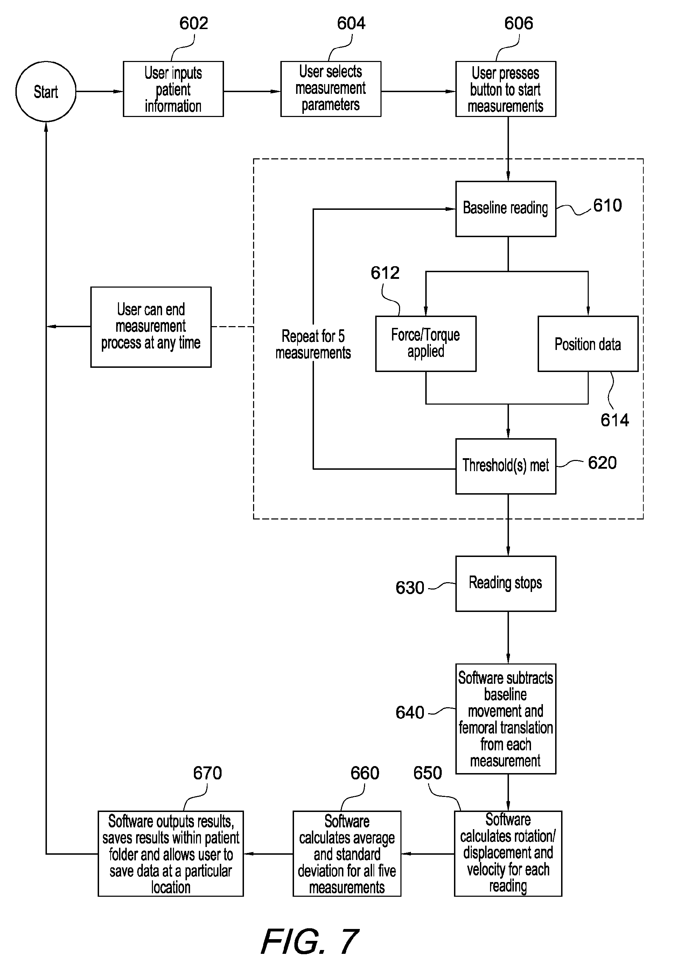

An exemplary system 100 is depicted in Figure 1. System 100 comprises a computer 110 with a display 112. Computer 110 has software that implements process 300 shown in Figure 6. Display 112 is attached to computer 110 and is discussed further with regard to Figure 7.

In system 100, computer 110 is in communication with movement sensor module 120 that has ports 124 for attaching movement sensor 122 and a transmitter 126. The module 120 detects and measures movement in the X, Y, and Z direction of the movement sensor 122 using the transmitter 126. Movement sensor module 120 may also record the amount of time taken to move from a first location to a second location to allow the velocity and acceleration of movement in the X, Y, and Z directions to be calculated. Movement sensor module 120 also detects and measures changes in pitch, yawl, and roll of the movement sensor 122 using the transmitter 126. Movement sensor module 120 may also record the amount of time taken to move from a first position to a second position to allow the angular velocity and acceleration of movement sensor 122 to be calculated.

In an exemplary embodiment, movement sensor module 120 may be an Ascension 3D Guidance trakStar Magnetic Tracking System. In another embodiment, another type of tracking system that uses RFID or infrared cameras may be used. Computer 110 and movement sensor module 120 may communicate through a cable. Alternatively, computer 110 and movement sensor module 120 may communicate wirelessly.

Computer 110 is also in communication with force sensor module 130. Module 130 is attached to drawer force sensor 132 and rotational torque sensor 134. System 100 further includes boot 140. Rotational torque sensor 134 is attached to the bottom of boot 140. Attached to rotational torque sensor 134 is rotational handle 144. In the preferred embodiment, drawer force sensor 132 is attached to the top anterior portion of boot 140. Alternatively, drawer force sensor 132 may be attached to the top posterior portion of boot 140. Attached to drawer force sensor 132 is drawer handle 142. Boot 140 further comprises bootstraps 146. In the preferred embodiment, boot 140 may be an Aircast® Foam Walker boot fitted with inflatable air cells. Other types of boots 140 may also be used with system 100. In the preferred embodiment, computer 110 and force sensor module 130 communicate through a cable. Alternatively, computer 110 and force sensor module 130 may communicate wirelessly.

Figure 1 also shows a boot 140 placed on a leg 150 of a patient and held tightly against leg 150 using bootstraps 146. Boot 140 covers the tibia portion of leg 150 and should be tight enough around leg 150 so that forces applied to boot 140 will be transferred to and act on leg 150. Boot 140 may also include air pockets that may be inflated to secure boot 140 to leg 150. Alternatively, boot 140 may be lined with foam or other cushioning medium so that the boot 140 can be tightened without being uncomfortable on leg 150. Movement sensor 122 is attached to leg 150 to record any movements of leg 150. In other embodiments, more than one movement sensor 122 may be attached to leg 150. For example, a movement sensor 122 may be attached to the tibial portion of the leg 150 and to the femoral portion of the leg 150. The movement sensor 122 attached to the femoral portion of the leg 150 may be attached to the bony landmark of the distal femur. In Figure 1, leg 150 is shown having a 90 degree angle between the femur and tibia part of leg 150.

System 100 functions to record the movements of leg 150 using sensor 122 when a force is applied to drawer handle 142 and/or rotational handle 144. For example, when a rotation torque is applied to rotational handle 144 in a direction as indicated by rotational arrow 160 of Figure 1, rotational torque sensor 134 senses the torque and transmits a signal to force sensor module 130. Force sensor module 130 reads the signal and sends another signal indicating the amount of rotational torque being applied to rotational handle 144 to computer 110. The amount of torque may then be displayed on display 112. As torque is applied to rotational handle 144, the torque is transmitted to boot 140 and then to leg 150. As a result, a rotational force is applied to leg 150, particularly to the tibia portion of leg 150 since it is secured within boot 140.

Any movement of the tibial portion of the leg 150 is detected by movement sensor module 120. Movement sensor module 120 detects the movements of leg 150 by detecting a change in location of movement sensor 122 within the magnetic field output by transmitter 126. Movement sensor module 120 communicates the change in location of movement sensor 122 to computer 110. The movement may be displayed on display 112 as rotational movement or as another type of movement. Movement of leg 150 is also detected in a similar manner if torque is applied in a direction opposite rotational arrow 160.

Similarly, when an anterior force is applied to drawer handle 142 in a direction as indicated by drawer arrow 162, drawer force sensor 132 senses the forces and transmits a signal to force sensor module 130. Force sensor module 130 reads the signal and sends another signal indicating the amount of drawer force being applied to computer 110. The amount of force may then be displayed on display 112. As force is applied to drawer handle 142, the force is transmitted to boot 140 and then to leg 150. As a result, a drawer force is applied to leg 150, particularly to the tibial portion of leg 150 since it is secured within boot 140. Any movement of the tibial portion of the leg 150 is detected by movement sensor 122 and sent to movement sensor module 120. Movement sensor module 120 communicates the change in location of movement sensor 122 to computer 110. The movement may be displayed on display 112 as drawer movement or as another type of movement. Movement of leg 150 is also detected in a similar manner if a posterior force is applied in a direction opposite drawer arrow 162.

In system 100, force sensor module 130 and movement sensor module 120 are in communication with a computer 110, thus allowing information from modules 120 and 130 to be used together. Computer 110 also allows movement sensor module 120 and force sensor module 130 to communicate.

System 100 only shows movement sensor 122 being deployed. However, additional movement sensors may be attached to movement sensor module 120 through ports 124. Additional sensors may be placed on the femoral part of leg 150 or boot 140. Also, additional sensors may be placed on leg 150 near movement sensor 122.

Figure 2 is a side view of boot 140 with leg 150 extended according to an exemplary embodiment. Figure 2 shows movement sensor 122 attached to leg 150 by sensor strap 148. This allows movement sensor 122 to be quickly disconnected from leg 150 and connected to another leg or used for another procedure. Figure 2 also shows drawer force sensor 132, drawer handle 142, rotational torque sensor 134, and rotational handle 144 connected to boot 140.

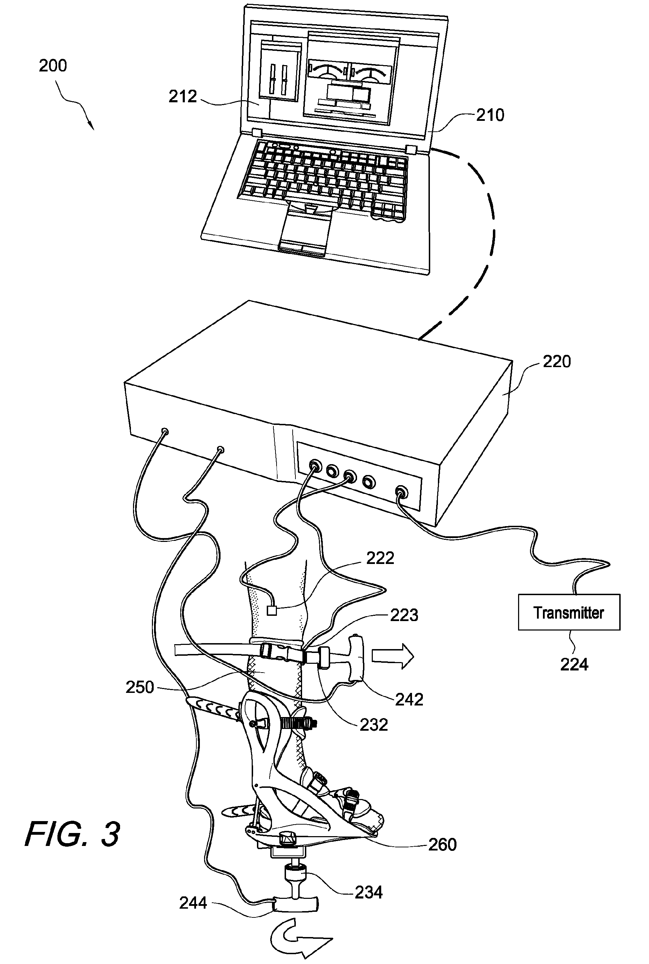

Figure 3 illustrates system 200 for measuring laxity in a joint according to an exemplary embodiment. System 200 comprises computer 110 with display 112 that communicates with console 220. Computer 110 and console 220 may communicate through a cable or wirelessly. Console 220 communicates with femoral movement sensor 222, tibial movement sensor 223, and transmitter 224. Console 220 detects and measures movement in the X, Y, and Z directions and changes in pitch, yawl, and roll of the femoral movement sensor 222 and tibial movement sensor 223 individually using the transmitter 224. Console 220 is further able to calculate velocity and acceleration in the X, Y, and Z directions and angular velocity and acceleration for each sensor 222, 223.

In this embodiment, console 220 is an Ascension 3D Guidance DriveBay Magnetic Tracking System and may accommodate up to four movement sensors. In another embodiment, another type of tracking system that uses RFID or infrared cameras may be used. In yet another embodiment, console 220 may accommodate more or less than four movement sensors. In yet another embodiment, system 200 may not include computer 110. In yet another embodiment, console 220 may include a display that displays information collected by console 220 and may process data collected by console 220 and send information to a remote location for storage.

The console 220 further communicates with drawer force sensor 232, drawer handle 242, rotational torque sensor 234, and rotational handle 244. The drawer handle 242 and rotational handle 244 operate to apply drawer and rotational forces respectively to leg 250. Drawer force sensor 232 and rotational torque sensor 234 measure the forces applied to leg 250. Sensors 232, 234 are strain gauges that are excited by current supplied by console 220 and send raw data to console 220 for conditioning, amplification, and/or processing.

Drawer handle 242 is attached directly to leg 250. Rotational handle 244 is coupled to boot 260, which is attached to leg 250. Tibial movement sensor 223 is placed on an upper portion of a tibia of leg 250 near the drawer handle 242 and femoral movement sensor 222 is placed on a femoral portion of leg 250, specifically on a bony landmark of the distal femur. Movement sensors 222, 223 are attached to leg 250 using an adhesive, such as tape, and may easily be repositioned and removed.

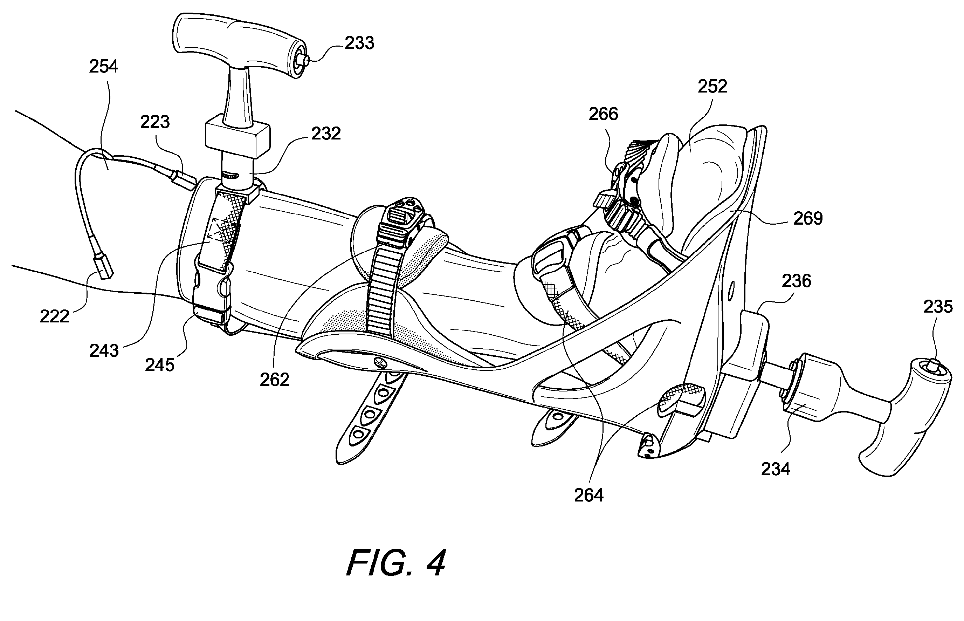

Figure 4 illustrates a side view of drawer and rotational handles 242, 244 and boot 260 according to an exemplary embodiment. Drawer handle 242 is attached directly to leg 250 using drawer strap 243 and buckle 245. Buckle 245 allows drawer handle 242 to be attached to and removed from leg 250. Drawer handle 242 further includes a control button 233 that communicates with console 220. Rotational handle 244 is attached to boot 260 using quick connect load cell 236 and also includes a control button 235 that communicates with console 220. Using quick connect load cell 236, rotational handle 244 may be easily transferred from one boot 260 to another boot 260.

With movement sensors 222, 223, rotational and drawer handles 242, 244 and rotational torque and drawer force sensors 232, 234 easily attached and detached from boot 260 and leg 250, a patient may be fitted with two boots and have laxity in both knees quickly checked by a doctor using a single system 200 by transferring the components quickly from one boot and leg to another boot and leg.

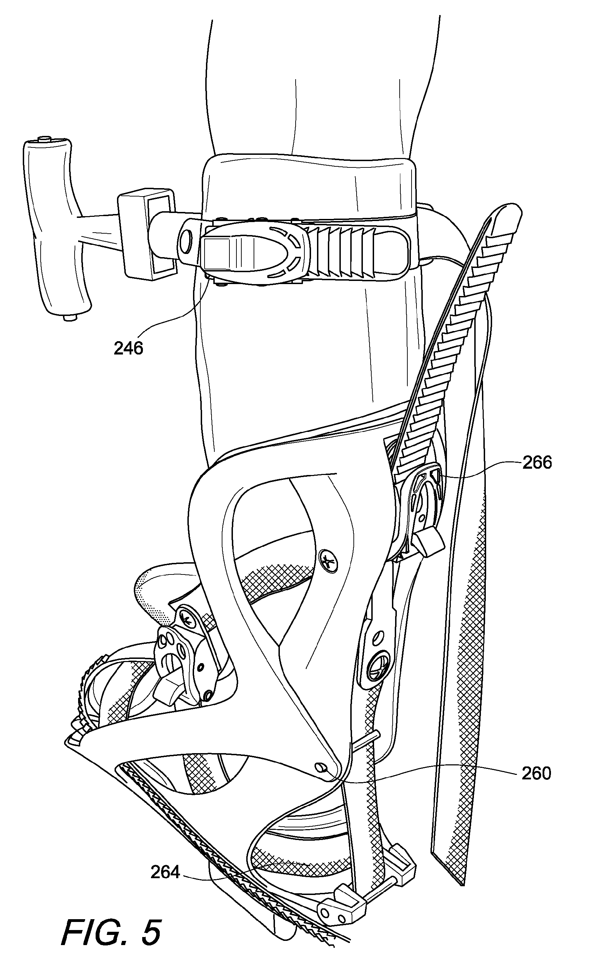

Boot 260 is a plastic modeled boot that is designed to accommodate many different sizes of legs 250. Boot 260 includes adjustable ladder ratchet straps 262, 266. Strap 262 runs across the tibial portion of leg 250 between knee 254 and foot 252. Strap 262 may be completely released to allow leg 250 to be placed within boot 250 without having to pass between the strap 262 and boot 250. Strap 262 may also be adjusted to accommodate varying sizes of legs. Strap 266 runs across foot 252 near the toes of foot 252. Strap 266 may be adjusted to accommodate varying sizes of feet. Boot 260 further includes adjustable strap 264 between straps 262, 266 that crosses over the top and heel of foot 252 to prevent translation of leg 250 within boot 260. Strap 264 is adjusted using ladder ratchet 266 as illustrated in Figure 5.

Padding 269 is provided between the straps 262, 264, 266 and leg 250 and between boot 260 and leg 250. In another embodiment, boot 260 may be metal, polymer, or ceramic and may be come in various sizes to accommodate legs 250 of various sizes. For example, boot 260 may be sized accordingly to a shoe size of a patient.

Figure 5 illustrates a back view of drawer strap 243 and boot 260. Drawer strap 243 includes a drawer force ladder ratchet 246 that allows the drawer handle 242 to be adjustably attached to leg 250. Drawer force ladder ratchet 246 and buckle 245 allow drawer strap 243 to be easily removed so that drawer handle 242 and drawer force sensor 232 may be easily repositioned along leg 250 or moved to another leg. Boot 260 further includes ladder ratchet 266 coupled to strap 264 for adjusting the length of strap 264 and tightening strap 264 against leg 250.

In use, force is applied to leg 250 using drawer and rotational handles 242, 244 individually or in combination. The force applied by the drawer and rotational handles 242, 244 is determined by the drawer force sensor 232 and rotational torque sensor 234 respectively. Console 220 detects movement of leg 250 using femoral and tibial movement sensors 222, 223. Specifically, femoral movement sensor 222 detects movement of the femoral portion of leg 250 and tibial movement sensor 223 detects movement of the tibial portion of leg 250. By detecting the movement of the femur and the tibia separately, compensation for femoral translation artifacts or movement is accomplished and true movement of the tibia and laxity in the knee joint may be better determined. Furthermore, the velocity of the movement sensors 222, 223 may be calculated. By tracking the velocity of the movement sensors 222, 223 more consistent readings may be obtained because measurements of knee laxity may change when a drawer or rotational force is applied inconsistently to leg 250 as manifest in by differing velocities of movement sensors 222, 223.

The console 220 may also determine the flexion angle between the femoral and tibial portions of leg 250 using femoral and tibial movement sensors 222, 223. At different flexion angles, the laxity in the knee joint of the leg 250 may change. To ensure consistency in flexion angles when laxity of the knee joint is measured, the user designates a flexion angle and the flexion angle is calculated using movement sensors 222, 223. In some embodiment, if the calculated flexion angle deviates from the set flexion angle the measurement may be disregarded.

Once information is collected by sensors 222, 223, 232, and 234 and sent to console 220, console 220 may process the information or may send the information to computer 210 for processing. Console 220 may also send processed information to computer 210 for storage.

Figure 6 is a flowchart showing process 300 using system 100 to measure laxity in a joint according to an exemplary embodiment. First, leg 150 of a patient is placed in the desired testing position, the air cells in boot 140 are inflated to create a snug, but comfortable fit for the patient. Movement sensor 122 is attached to leg 150.

Next, in step 302, the user selects measurement parameters. Process 300 allows a user to select an amount of rotational torque or drawer force that is applied before movement sensor module 120 captures and reads movement from movement sensor 122. For example, the user may desire that a drawer laxity of a joint be measured when 20 lbs of drawer force are applied to the joint. The user would select the measurement parameter of 20 lbs during step 302. With this selection, when the user applies 20 lbs of drawer pressure to the joint, process 300 automatically determines movement of the joint using movement sensor 122 and computer 110. In another embodiment, process 300 may use predefined drawer force and/or rotational torque values. In yet another embodiment, no amount of rotational torque or drawer force may be selected. System 100 may just track the maximum drawer or rotational torque force applied to leg 150. The movement of the joint may be shown on display 112.

During step 302, the user may select measurement parameters for rotational laxity only. The user may select the prescribed counterclockwise torque and/or the clockwise torque, such as approximately 4 N/m to measure the angular range of motion of the joint. The prescribed torque may range between 0 N/m and 15 N/m. The user may also select measurement parameters for drawer laxity and measure only drawer displacement. As another option, the user may select a combination of measurement parameters for both rotational and drawer laxity. Additionally, for each selection of measurement parameters, the user may select to use max force or less than the max force.

Further, during step 302, the user may designate the angle between the tibia portion and the femur portion of the leg when the measurement is taken. For example, the user may select a flexion angle of 30, 45, 60, or 90 degrees.

Also, in step 302, the user may select the condition of the leg 150 to be tested. For example, the user could select to test the injured knee or the non-injured knee.

Next, in step 304, user commences taking measurements by sending a signal to computer 110. The signal may be sent by pushing a button on a periphery of computer 110 or through pushing a button on display 112.

Further, in step 310, process 300 takes baseline readings. Baseline readings are taken by determining the present location of movement sensor 122. Process 300 may also determine the current force on drawer force sensor 132 and the current torque on rotational torque sensor 134. Process 300 uses these values as baseline values. During step 310, process 300 may take more than one baseline reading before proceeding to step 320.

Next, in step 320, computer 110 receives inputs from force sensor module 130. The user applies a torque and/or drawer force on boot 140 using rotational handle 144 and/or drawer handle 142. Based on the user-selected measurements set in step 302, once a certain torque or force threshold is met in step 322, computer 110 captures data from movement sensor module 120 indicating the present location of movement sensor 122. Steps 320 and 322 are repeated five times to capture five measurements. In other embodiments, steps 320 and 322 may be done only once or many times. The user may end process 300 at any time during steps 320 and 322.

After completing all measurements in steps 320 and 322, in step 340, computer 110 subtracts each measurement captured in step 322 from the baseline measurement taken during step 310.

Next, in step 350, computer 110 calculates the rotational movement (i.e. range of motion) or drawer movement (i.e. displacement) of the joint for each measurement that was captured. As part of calculating the movement of the joint, computer 110 may substrate femoral translational movement artifacts. The computer 110 may also calculate the average velocity of the movement sensor 122 and the flexion angle of the leg 150 for each measurement captured.

Further, in step 360, computer 110 calculates the average and standard deviation for all the measurements taken during steps 322. Computer 110 may disregard measurements taken where the average velocity or flexion angle was outside a set parameters or may highlight these measurements to indicate that they may not be accurate. In step 370, computer 110 allows the user to output the results and save the data in a desired format, such as a CSV file. Computer 110 may also generate patient folders to maintain patient information to allow the patient information, such as patient demographics and history, to be accessed at a later time.

It should be understood, that process 300 as described above is not limiting. Additional steps may be performed at any time during process 300 or the described steps may be altered or not performed or the steps may not be performed in the sequence outlined above. For example, in another embodiment, step 322 is performed continuously so that continuous measurements of movement sensor 122 are taken as a force or torque is applied to boot 140. In yet another embodiment, steps 340 and 350 are done as every measurement is taken in step 322. This allows the measurements of rotational and drawer laxity to be displayed immediately after they are taken.

Figure 7 is a flowchart showing process 600 that uses system 200 to measure laxity in a joint according to an exemplary embodiment. To begin process 600, leg 250 of a patient is placed in boot 260 and ratchet straps 262, 266 and strap 264 are adjusted to secure boot 260 to leg 250 so that rotational force exerted on boot 260 transfers to leg 250 without undue losses. Drawer handle 242 is attached directly to leg 250 using drawer strap 243 and buckle 245 and adjusted using drawer force ladder ratchet 246. Drawer strap 243 is adjusted so that drawer handle is snug against leg 250 and able to readily transfer force applied to drawer handle 242 to leg 250. Movement sensors 222, 223 are placed on the femoral and tibial portions of leg 250 respectively.

During step 602, the user inputs patient information, such as the patient's name, id number, age, relevant health conditions, or any other relevant information. In step 604, the user may input measurement parameters, such as a drawer and/or rotational force threshold. In step 606, the user commences taking measurements by sending a signal to computer 210 or console 220. The signal may be sent by pushing a button on a periphery of computer 210, pushing a button on display 212, pushing a button on console 220, pushing control button 233 on drawer handle 242, or pushing control button 235 on rotational handle 244.

Next, in step 610, system 200 takes baseline readings of the drawer force and rotational torque using drawer force sensor 232 and rotational torque sensor 234 respectively. System 200 also determines the baseline positions of movements sensors 222, 223. In step 612, force and/or torque is applied to leg 250 through the drawer and/or rotational handles 142, 144. As force and/or torque is applied to leg 250, in step 614 the current positions of movements sensors 222, 223 are determined. In step 620, once the drawer force and/or rotational torque applied to leg 250 is greater than the threshold input in step 604, system 200 determines the current positions of movements sensors 222, 223. Steps 610, 612, 614, and 620 are repeated 5 times. In step 630, force is no longer applied to leg 250 and determination of the position of movements sensors 222, 223 is stopped.

Steps 640-670 of process 600 are then performed in a similar manner as described above with respect to steps 340-370 of process 300.

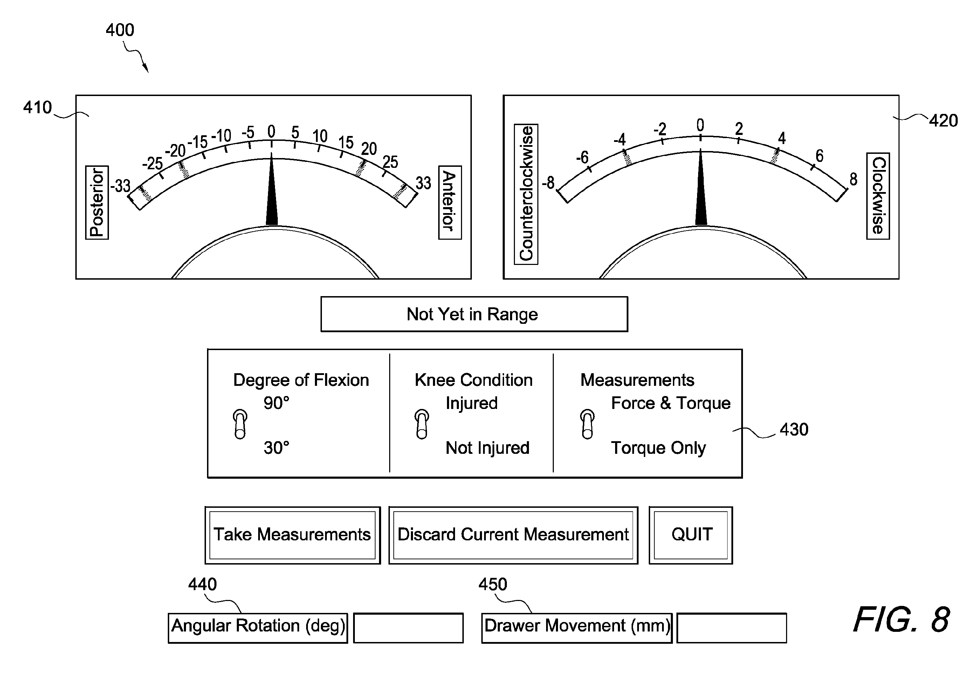

Figure 8 shows screen 400 that may be shown on displays112, 212 according to an exemplary embodiment. Screen 400 shows drawer force gauge 410 and rotational torque gauge 420. Drawer force gauge 410 shows the amount of force being sensed by drawer force sensor 132, 232 in either the anterior or the posterior direction. Rotational torque gauge 420 shows the torque being sensed by rotational torque sensor 134, 234 in either the clockwise or the counterclockwise direction. Rotational torque gauge 420 and drawer force gauge 410 show the real-time values of rotational torque sensor 134, 234 and drawer force sensor 132, 232.

Screen 400 also shows various options in option setter 430 that may be selected including whether the patient's leg 150, 250 has a 90 or 30 degree angle between the femur and tibia. Further, screen 400 displays the angular movement of the joint in angular rotation output box 440. Screen 400 also displays the displacement of the joint in drawer movement output box 450.

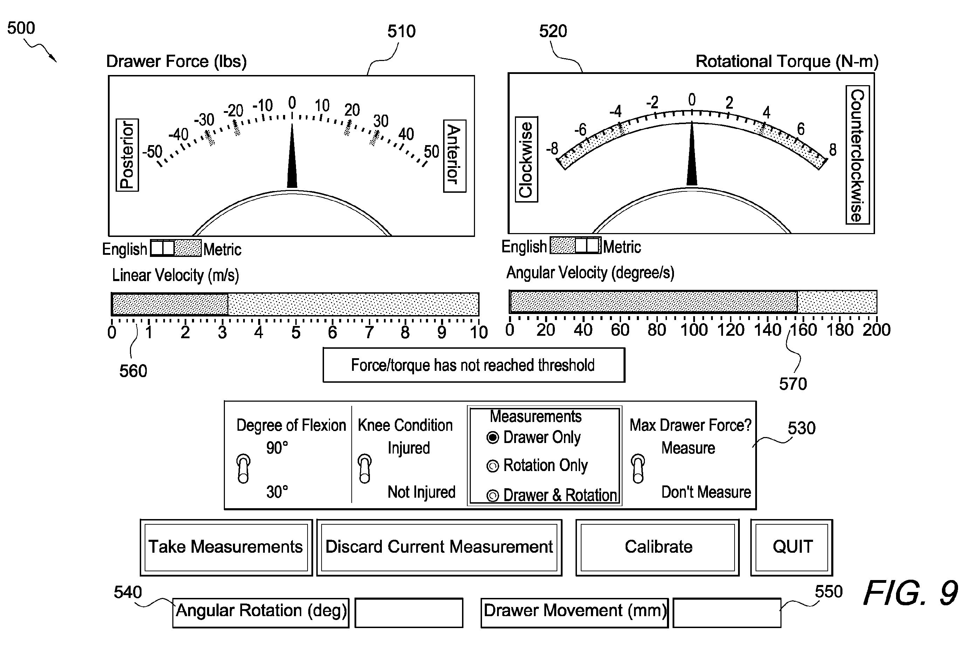

Figure 9 shows screen 500 that may be shown on displays 112, 212 according to an exemplary embodiment. Screen 500 has various options in option setter 530 that may be selected including the flexion angle of the patient's leg 150, 250, the condition of the knee, and the type of measurement to be taken, such as, torque force, drawer force, or torque and drawer force. Screen 500 further displays the angular movement of the joint in angular rotation output box 540 and the displacement of the joint in drawer movement output box 550. Screen 500 also display the calculated linear and angular velocities of the movement sensors 122, 222, 223 in respective linear and angular output bars 560, 570. Furthermore, screen 500 displays the drawer force and rotational torque applied to leg 150, 250 in respective drawer force gauge 510 and rotational force gauge 520.

Although the present invention has been described in relation to particular embodiments thereof, many other variations and modifications and other uses will become apparent to those skilled in the art. For example, system 100 and process 300 may be applied to other joints, such as the shoulder or ankle, to determine rotational and drawer laxity.

## Claims

1. A system for determining laxity in a joint, the system including:
at least one force sensor for measuring an amount of force applied to an appendage coupled to the joint;
at least one movement sensor for measuring movement in the appendage when the force is applied to the appendage; and
a system controller connected to the force sensor and movement sensor, wherein the force sensor and the movement sensor communicate through the system controller.

2. The system of claim 1, wherein the at least one force sensor is a drawer force sensor for determining an amount of drawer force applied to the appendage of the joint.

3. The system of claim 2, further comprising a rotational force sensor for determining an amount of rotational force applied to the appendage of the joint.

4. The system of claim 1, wherein the joint is a shoulder joint.

5. The system of claim 1, wherein the joint is an ankle joint.

6. The system of claim 1, wherein the joint is a knee joint.

7. The system of claim 6, the system further comprising a boot that houses the appendage, wherein the appendage is a foot and lower leg of a patient.

8. The system of claim 7, wherein the at least one movement sensor is removably coupled to the boot.

9. The system of claim 1, wherein the at least one movement sensor detects movement when the force applied to the appendage, as determined by the force sensor, exceeds a threshold.

10. The system of claim 1, wherein the at least one movement sensor detects movement in six degrees of freedom.
